# EUROPEAN PATENT APPLICATION

(11) **EP 3 932 962 A1**
(43) Date of publication of application: **05.01.2022**
(21) Application number: 20765479.9
(22) Date of filing: 27.02.2020
(51) Int. Cl.: C08F 220/06, C08L 33/02, C08F 2/30

(54) **AQUEOUS POLYMER DISPERSION COMPOSITION**

(30) Priority: 01.03.2019 JP 2019037991
(71) Applicant: Sumitomo Seika Chemicals Co., Ltd., Kako-gun, Hyogo 675-0145 (JP)
(72) Inventor: MURAKAMI, Ryosuke, Himeji-shi, Hyogo 672-8076 (JP); YAMAGUCHI, Hirofumi, Himeji-shi, Hyogo 672-8076 (JP); UEZUMI, Chiaki, Himeji-shi, Hyogo 672-8076 (JP)
(74) Representative: Vossius & Partner Patentanwälte Rechtsanwälte mbB
(86) International application number: PCT/JP2020/007970
(87) International publication number: WO 2020/179602

(57) **Abstract**

Provided is an aqueous dispersion composition that comprises a carboxyl-group-containing water-soluble copolymer in a relatively high concentration, but has intact handleability. More specifically, provided is, for example, an aqueous dispersion composition comprising 4 to 8 mass% of a copolymer obtained by polymerization in the presence of a nonionic surfactant with one or more polyoxyethylene chains, the copolymer being at least one member selected from the group consisting of
(1) copolymers obtained by polymerizing the following (i) and (ii),
(2) copolymers obtained by polymerizing the following (i) and (iii), and
(3) copolymers obtained by polymerizing the following (i), (ii), and (iii):
(i) 100 parts by mass of a (meth)acrylic acid;
(ii) 0.001 to 1 part by mass of a compound with two or more ethylenically unsaturated groups; and
(iii) 0.5 to 5 parts by mass of a (meth)acrylic acid alkyl ester having an alkyl group with 10 to 30 carbon atoms.

## Description

### Technical Field

The present disclosure relates to, for example, a composition in which a polymer (in particular, a carboxyl-group-containing water-soluble copolymer) is dispersed in water.

### Background Art

Water-soluble copolymers containing carboxyl groups are used in various fields as a thickening agent for cosmetics etc., a moisturizer for poultices etc., a suspension stabilizer for emulsions, suspensions, etc., and a gelling base for batteries etc.

### Citation List

### Patent Literature

PTL 1: WO 2014/021434

### Summary of Invention

### Technical Problem

Carboxyl-group-containing water-soluble copolymers have a thickening effect. By dissolving or dispersing a carboxyl-group-containing water-soluble copolymer in water etc., a viscous composition can be obtained. The thickening effect drastically increases with an increase in the amount of the copolymer; thus, water that contains a large amount of the copolymer has an overly high viscosity or is in the form of a nearly solid, and has significantly reduced handleability. Therefore, an aqueous dispersion composition with a concentration of 1 to 1.5 mass%, or about 2 mass% at most, is typically prepared for use in the production of subsequent products (e.g., cosmetics).

On the other hand, a higher concentration of an aqueous dispersion composition obtained by dispersing the copolymer in water increases efficiency more in the production of products such as cosmetics. Therefore, it would be advantageous if it is possible to prepare an aqueous dispersion composition that comprises the copolymer in a high concentration, but has intact handleability.

### Solution to Problem

The present inventors found the possibility that a carboxyl-group-containing water-soluble copolymer obtained by polymerizing specific components in the presence of a specific nonionic surfactant can produce an aqueous dispersion composition with maintained handleability, even when the copolymer is dispersed at a relatively high concentration in water. Based on this finding, the inventors made further improvements.

The present disclosure encompasses, for example, the subject matter described in the following items.

### Item 1.

An aqueous dispersion composition comprising 4 to 8 mass% of a copolymer obtained by polymerization in the presence of a nonionic surfactant with one or more polyoxyethylene chains, the copolymer being at least one member selected from the group consisting of
(1) copolymers obtained by polymerizing the following (i) and (ii),
(2) copolymers obtained by polymerizing the following (i) and (iii), and
(3) copolymers obtained by polymerizing the following (i), (ii), and (iii):
   (i) 100 parts by mass of a (meth)acrylic acid;
   (ii) 0.001 to 1 part by mass of a compound with two or more ethylenically unsaturated groups; and
   (iii) 0.5 to 5 parts by mass of a (meth)acrylic acid alkyl ester having an alkyl group with 10 to 30 carbon atoms.

### Item 2.

The aqueous dispersion composition according to Item 1, wherein the copolymer is obtained by polymerization in the presence of a nonionic surfactant with one or more polyoxyethylene chains in an amount of 0.5 to 10 parts by mass per 100 parts by mass of the (meth)acrylic acid (i).

### Item 3.

The aqueous dispersion composition according to Item 1 or 2, having a viscosity of 60000 mPa·s or less.

### Item 4.

The aqueous dispersion composition according to any one of Items 1 to 3, wherein the nonionic surfactant with one or more polyoxyethylene chains is selected from the group consisting of polyhydric alcohol fatty acid ester-ethylene oxide adducts, block copolymers of hydroxy fatty acids and ethylene oxide, and polyoxyethylene castor oil.

### Item 5.

An aqueous dispersion composition comprising 4 to 8 mass% of at least one member selected from the group consisting of
(1) copolymers obtained by polymerizing the following (i) and (ii),
(2) copolymers obtained by polymerizing the following (i) and (iii), and
(3) copolymers obtained by polymerizing the following (i), (ii), and (iii), and having a viscosity of 60000 mPa·s or less:
   (i) 100 parts by mass of a (meth)acrylic acid;
   (ii) 0.001 to 1 part by mass of a compound with two or more ethylenically unsaturated groups; and
   (iii) 0.5 to 5 parts by mass of a (meth)acrylic acid alkyl ester having an alkyl group with 10 to 30 carbon atoms.

### Item 6.

The aqueous dispersion composition according to any one of Items 1 to 5, wherein the (meth)acrylic acid alkyl ester (iii) is a (meth)acrylic acid alkyl ester having an alkyl group with 18 to 24 carbon atoms.

### Item 7.

The aqueous dispersion composition according to any one of Items 1 to 6, wherein the compound with two or more ethylenically unsaturated groups (ii) is at least one compound selected from the group consisting of pentaerythritol allyl ether, tetraallyloxyethane, triallyl phosphate, and polyallylsaccharose.

### Advantageous Effects of Invention

Provided is an aqueous dispersion composition of a carboxyl-group-containing water-soluble copolymer that can achieve maintained handleability, even when dispersed at a relatively high concentration in water.

### Description of Embodiments

Embodiments encompassed by the present disclosure are described in more detail below. The present disclosure preferably encompasses, for example, a viscous composition (in particular, an aqueous dispersion composition of a carboxyl-group-containing water-soluble copolymer that can achieve maintained handleability), and a method for preparing the viscous composition. However, the present disclosure is not limited thereto and encompasses everything disclosed in this specification and recognizable to those skilled in the art.

The present disclosure encompasses an aqueous dispersion composition comprising 4 to 8 mass% of a copolymer obtained by polymerization in the presence of a nonionic surfactant with one or more polyoxyethylene chains, the copolymer being at least one member selected from the group consisting of
(1) copolymers obtained by polymerizing the following (i) and (ii),
(2) copolymers obtained by polymerizing the following (i) and (iii), and
(3) copolymers obtained by polymerizing the following (i), (ii), and (iii):
   (i) 100 parts by mass of a (meth)acrylic acid;
   (ii) 0.001 to 1 part by mass of a compound with two or more ethylenically unsaturated groups; and
   (iii) 0.5 to 5 parts by mass of a (meth)acrylic acid alkyl ester having an alkyl group with 10 to 30 carbon atoms.

The present disclosure also encompasses an aqueous dispersion composition comprising 4 to 8 mass% of at least one member selected from the group consisting of
(1) copolymers obtained by polymerizing (i) and (ii) above,
(2) copolymers obtained by polymerizing (i) and (iii) above, and
(3) copolymers obtained by polymerizing (i), (ii), and (iii) above, and having a viscosity of 60000 mPa·s or less.

In this specification, the aqueous dispersion composition of polymer encompassed by the present disclosure is sometimes referred to as "the aqueous dispersion composition of the present disclosure." The copolymers (1), (2), and (3) above encompassed by the present disclosure may also be referred to as "the copolymer (1)," "the copolymer (2)," and "the copolymer (3)," respectively. Additionally, the copolymers (1) to (3) may also be referred to collectively as "the copolymers of the present disclosure." The copolymers of the present disclosure are more preferably, but are not particularly limited to, (1) or (3), and particularly preferably (3).

As stated above, the copolymer (3) is obtained by polymerizing 100 parts by mass of a (meth)acrylic acid, 0.001 to 1 part by mass of a compound with two or more ethylenically unsaturated groups, and 0.5 to 5 parts by mass of a (meth)acrylic acid alkyl ester having an alkyl group with 10 to 30 carbon atoms.

In this specification, the term "(meth)acrylic" means acrylic and/or methacrylic. For the (meth)acrylic acid, either acrylic acid or methacrylic acid may be used alone, or used in combination.

The (meth)acrylic acid alkyl ester having an alkyl group with 10 to 30 (10, 11, 12, 13, 14, 15, 16, 17, 18, 19, 20, 21, 22, 23, 24, 25, 26, 27, 28, 29, or 30) carbon atoms refers to an ester of (meth)acrylic acid and a higher alcohol having an alkyl group with 10 to 30 carbon atoms. The carbon number of this alkyl group is more preferably, for example, 12 to 30, 14 to 28, 16 to 26, or 18 to 24. Examples of the (meth)acrylic acid alkyl ester include, but are not particularly limited to, esters of (meth)acrylic acid with stearyl alcohol, esters of (meth)acrylic acid with eicosanol, esters of (meth)acrylic acid with behenyl alcohol, and esters of (meth)acrylic acid with tetracosanol. Among these (meth)acrylic acid alkyl esters, stearyl methacrylate, eicosanyl methacrylate, behenyl methacrylate, and tetracosanyl methacrylate are preferred. These (meth)acrylic acid alkyl esters may be used alone, or in a combination of two or more. These (meth)acrylic acid alkyl esters for use may be commercial products, such as BLEMMER VMA-70, product name, produced by NOF Corporation.

The amount of the (meth)acrylic acid alkyl ester for use is 0.5 to 5 parts by mass, preferably 0.5 to 4 parts by mass or 1 to 4 parts by mass, and more preferably 1 to 3 parts by mass, per 100 parts by mass of the (meth)acrylic acid.

Examples of the compound with two or more ethylenically unsaturated groups include but are not particularly limited to, a compound in which a polyol, such as ethylene glycol, propylene glycol, polyoxyethylene glycol, polyoxypropylene glycol, glycerol, polyglycerol, trimethylolpropane, pentaerythritol, saccharose, or sorbitol is substituted with two or more acrylic acid esters; a compound in which a polyol mentioned above is substituted with two or more allyl ethers; and diallyl phthalate, triallyl phosphate, allyl methacrylate, tetraallyloxyethane, triallyl cyanurate, divinyl adipate, vinyl crotonate, 1,5-hexadiene, divinyl benzene, and polyallylsaccharose. Among these, the compound with two or more ethylenically unsaturated groups is preferably pentaerythritol allyl ether (more preferably pentaerythritol triallyl ether or pentaerythritol tetraallyl ether), tetraallyloxyethane, triallyl phosphate, or polyallylsaccharose, since the use of a small amount of the resulting alkyl-modified carboxyl-group-containing water-soluble copolymer can produce a viscous composition with high thickening properties, and since high suspension stability can be imparted to emulsions, suspensions, etc. These compounds with two or more ethylenically unsaturated groups may be used alone, or in a combination of two or more.

The amount of the compound with two or more ethylenically unsaturated groups for use is 0.001 to 1 part by mass per 100 parts by mass of the (meth)acrylic acid. The lower limit of this range may be, for example, 0.005, 0.01, 0.015, 0.02, 0.025, 0.03, 0.035, or 0.04. The upper limit of this range may be, for example, 0.95, 0.9, 0.85, 0.8, 0.75, 0.7, 0.65, or 0.6. For example, the amount for use is preferably 0.01 to 0.8 parts by mass, and more preferably 0.02 to 0.7 parts by mass, per 100 parts by mass of the (meth)acrylic acid.

In the production of the copolymers of the present disclosure, the method of polymerizing (i) and (ii), or (i) and (iii), or (i), (ii), and (iii), is not particularly limited. Examples include a method of polymerizing these in a polymerization solvent in the presence of a radical polymerization initiator.

Examples of the radical polymerization initiator include, but are not particularly limited to, α,α'-azobisisobutyronitrile, 2,2'-azobis-2,4-dimethylvaleronitrile, 2,2'-azobis methyl isobutyrate, benzoyl peroxide, lauroyl peroxide, cumene hydroperoxide, and tertiary butyl hydroperoxide. These radical polymerization initiators may be used alone, or in a combination of two or more.

The amount of the radical polymerization initiator for use is not particularly limited and is preferably, for example, 0.01 to 0.45 parts by mass, and more preferably 0.01 to 0.35 parts by mass, per 100 parts by mass of the (meth)acrylic acid. When the radical polymerization initiator is used in this range, the polymerization reaction speed can be more appropriately controlled, making it possible to economically produce an alkyl-modified carboxyl-group-containing water-soluble copolymer.

The description for the copolymer (1) and the description for the copolymer (3) are in common except for the presence of (iii) above during polymerization, unless otherwise noted. Further, the description for the copolymer (2) and the description for the copolymer (3) are in common except for the presence of (ii) above during polymerization, unless otherwise noted. The description for the copolymer (3) in which (iii) above is not used corresponds to the description for the copolymer (1), while the description for the copolymer (3) in which (ii) above is not used corresponds to the description for the copolymer (2).

When obtained by polymerization in the presence of a nonionic surfactant with one or more polyoxyethylene chains, none of the copolymers (1) to (3) particularly increase the viscosity, even when they are dispersed at a high concentration (e.g., about 4 to 8 mass%) in water, and the handleability can be maintained. These copolymers can be obtained, for example, by adding the raw materials for the copolymers, as well as the surfactant, to a polymerization solvent, followed by polymerization. If the copolymers (1) to (3) (i.e., the copolymers obtained by polymerizing (i) and (ii) above, the copolymers obtained by polymerizing (i) and (iii) above, and the copolymers obtained by polymerizing (i), (ii), and (iii) above) are not obtained by polymerization in the presence of a nonionic surfactant with one or more polyoxyethylene chains, the handleability cannot be maintained when they are dispersed at a high concentration (e.g., about 4 to 8 mass%) in water.

Preferable examples of the nonionic surfactant with one or more polyoxyethylene chains include polyhydric alcohol fatty acid ester-ethylene oxide adducts, block copolymers of hydroxy fatty acids and ethylene oxide, and polyoxyethylene castor oil.

Preferable examples of the polyhydric alcohol fatty acid ester-ethylene oxide adducts include ester compounds of polyoxyethylene hydrogenated castor oil and polyhydric alcohol fatty acids. The polyhydric alcohol fatty acid here is preferably a saturated or unsaturated polyhydric (in particular, divalent) alcohol fatty acid with 14 to 24 (14, 15, 16, 17, 18, 19, 20, 21, 22, 23, or 24) carbon atoms. More specifically, for example, isopalmitic acid, isostearic acid, and isooleic acid are preferred. The average number of moles of ethylene oxide added to constitute polyoxyethylene in ester compounds of polyoxyethylene hydrogenated castor oil and polyhydric alcohol fatty acids is not particularly limited, and may be, for example, about 20 to 100 or about 30 to 70. Particularly preferable examples of the ester compounds of polyoxyethylene hydrogenated castor oil and polyhydric alcohol fatty acids include polyoxyethylene hydrogenated castor oil isostearates.

In polyoxyethylene castor oil, the number of moles of ethylene oxide added is preferably about 2 to 10, and more preferably about 2 to 5.

The block copolymer of a hydroxy fatty acid and ethylene oxide can be rephrased as a copolymer of a polyhydroxy fatty acid and polyoxyethylene. The fatty acid of polyhydroxy fatty acid is preferably a fatty acid with 14 to 22 carbon atoms. Preferable examples include myristic acid, palmitic acid, and stearic acid. The hydroxy fatty acid is preferably, for example, hydroxymyristic acid, hydroxypalmitic acid, or hydroxystearic acid, and particularly preferably hydroxystearic acid. The hydroxystearic acid is particularly preferably 12-hydroxystearic acid. The polyhydroxy fatty acid is particularly preferably a polyhydroxystearic acid. The block copolymer of a hydroxy fatty acid and ethylene oxide is particularly preferably a block copolymer of 12-hydroxystearic acid and ethylene oxide.

The nonionic surfactant with one or more polyoxyethylene chains can be used alone, or in a combination of two or more.

The amount of the nonionic surfactant with one or more polyoxyethylene chains for use is preferably about 0.5 to 10 parts by mass per 100 parts by mass of the (meth)acrylic acid (i). The lower limit of the range based on parts by mass may be, for example, about 0.75, 1.0, 1.25, or 1.5; and the upper limit of the range based on parts by mass may be, for example, about 9.5, 9, 8.5, 8, 7.5, 7, 6.5, 6, or 5.5. For example, the amount is more preferably about 1 to 7.5 parts by mass.

The polymerization solvent is not particularly limited, and is preferably a solvent that dissolves (meth)acrylic acid, the (meth)acrylic acid alkyl ester, and the compound with two or more ethylenically unsaturated groups, and that does not dissolve the resulting alkyl-modified carboxyl-group-containing water-soluble copolymer. Specific examples of such polymerization solvents include normal pentane, normal hexane, normal heptane, normal octane, isooctane, cyclopentane, methylcyclopentane, cyclohexane, methylcyclohexane, benzene, toluene, xylene, chlorobenzene, ethylene dichloride, ethyl acetate, isopropyl acetate, ethyl methyl ketone, and isobutyl methyl ketone. Among these polymerization solvents, ethylene dichloride, normal hexane, normal heptane, and ethyl acetate are preferred from the standpoint of stable quality and easy availability. These polymerization solvents may be used alone, or in a combination of two or more.

The amount of the polymerization solvent for use is not particularly limited, and is preferably, for example, 200 to 10,000 parts by mass, and more preferably 300 to 2,000 parts by mass, per 100 parts by mass of the (meth)acrylic acid. The use of the polymerization solvent within this range suppresses aggregation of the alkyl-modified carboxyl-group-containing water-soluble copolymer even when the polymerization reaction proceeds, allowing stirring to be performed uniformly, and the polymerization reaction to proceed efficiently.

The atmosphere during the polymerization reaction is not particularly limited as long as the polymerization reaction can proceed. Examples include an inert gas atmosphere, such as nitrogen gas or argon gas.

The reaction temperature during the polymerization reaction is not particularly limited as long as the polymerization reaction can proceed, and is preferably, for example, 50 to 90°C, and more preferably 55 to 75°C. When the polymerization reaction is performed within this reaction temperature range, an increase in the reaction solution viscosity can be suppressed, and the reaction can be easily controlled; additionally, the bulk density of the resulting alkyl-modified carboxyl-group-containing water-soluble copolymer can be controlled.

The reaction time for the polymerization reaction cannot be generalized because it varies depends on the reaction temperature. The reaction time is typically 2 to 10 hours.

After completion of the reaction, for example, the reaction solution is heated to 80 to 130°C, and the polymerization solvent is removed, whereby a white fine powder of an alkyl-modified carboxyl-group-containing water-soluble copolymer can be isolated.

In the aqueous dispersion composition of the present disclosure, the copolymers of the present disclosure may be used alone, or in a combination of two or more.

The aqueous dispersion composition of the present disclosure comprises about 4 to 8 mass% of the copolymers of the present disclosure. The lower limit of this range may be, for example, 4.5 or 5 mass%. The upper limit of this range may be 7.5 or 7 mass%. It is more preferably, for example, 4.5 to 7.5 mass%.

The viscous composition of the present disclosure preferably has a viscosity of 60000 mPa·s or less, more preferably 50000 mPa·s or less, and still more preferably 40000 mPa·s or less. The lower limit of the viscosity is preferably 1000 mPa·s or more, and more preferably 1500 mPa·s or more. The viscosity is measured at an ordinary temperature (25°C) using a Brookfield viscometer (model number: DV1MRVTJ0) at a rotation speed of 20 revolutions per minute. For rotors used in the measurement, rotor No. 3 is used for the viscosity of less than 2000 mPa·s, rotor No. 4 is used for the viscosity of 2000 mPa·s or more and less than 5000 mPa·s, rotor No. 5 is used for the viscosity of 5000 mPa·s or more and less than 15000 mPa·s, rotor No. 6 is used for the viscosity of 15000 mPa·s or more and less than 40000 mPa·s, and rotor No. 7 is used for the viscosity of 40000 mPa·s or more.

The aqueous dispersion composition of the present disclosure can be prepared, for example, by gradually adding the copolymers of the present disclosure little by little to water with stirring.

The aqueous dispersion composition of the present disclosure may consist only of the copolymers of the present disclosure and water, or may comprise other additives and active ingredients according to its application as long as the effects of the present disclosure are not impaired. For example, when used as cosmetics, the aqueous dispersion composition of the present disclosure may comprise a moisturizer, an antioxidant, a blood circulation stimulant, a cooling agent, an antiperspirant, a disinfectant, a skin activator, a deodorant, a surfactant, a fragrance, a dye, and the like. In particular, from the standpoint of eliminating stickiness to the skin and further improving the excellent feeling of smoothness, the aqueous dispersion composition of the present disclosure, when used for preparing cosmetics (e.g., concentrated liquid), preferably comprises, for example, a moisturizer, such as glycerol, propylene glycol, dipropylene glycol, 1,3-butylene glycol, polyethylene glycol, sorbitol, sodium lactate, sodium 2-pyrrolidone-5-carboxylate, sodium hyaluronate, and sodium acetylated hyaluronate.

When other additives and active ingredients are used to produce an aqueous dispersion composition of the present disclosure, these additives and active ingredients may also be added, for example, when the copolymers of the present disclosure are added to water as described above.

The aqueous dispersion composition of the present disclosure has the viscosity mentioned above and is in the form of, for example, a viscous liquid, flowable gel, or cream. Thus, the aqueous dispersion composition of the present disclosure has excellent handleability despite the presence of a relatively high concentration of a carboxyl-group-containing water-soluble copolymer.

The aqueous dispersion composition of the present disclosure is used in the fields of, for example, cosmetics, pharmaceuticals (in particular, preparation for external use for skin), toiletry products, household products, and water-soluble paint. In particular, the aqueous dispersion composition of the present disclosure is used as a precursor (e.g., a concentrated liquid) for these products, or as a concentrated liquid etc. of a thickening liquid used for these products.

When the aqueous dispersion composition of the present disclosure or a diluted product of the aqueous dispersion composition of the present disclosure is used for cosmetics, the form of the formulation is not particularly limited. Examples include lotions, emulsions, serums, creams, cream pack, massage creams, hair-setting gels, sunscreens, styling gels, eyeliners, mascaras, lipsticks, and foundations. When the aqueous dispersion composition of the present disclosure or a diluted product of the aqueous dispersion composition of the present disclosure is used as a toiletry product, the form of the formulation is not particularly limited. Examples include cleansing creams, cleansing gels, facial cleansing foams, hair washes, body washes, and hair conditioners.

In this specification, the terms "comprising" and "containing" include "consisting essentially of" and "consisting of." Further, the present disclosure includes any combination of the constituent requirements described in this specification.

In addition, the various characteristics (properties, structures, functions, etc.) described in each embodiment of the present disclosure described above may be combined in any way in specifying the subjects included in the present disclosure. In other words, the present disclosure includes all the subjects comprising all combinations of the combinable characteristics described in this specification.

### Examples

The present disclosure is described in more detail below. However, the present disclosure is not limited to the following Examples.

### Measurement Methods

The viscosity of the viscous compositions obtained in Examples and Comparative Examples below were evaluated according to the following methods.

After immersing an evaluation sample (each viscous composition) for 60 minutes or more in a constant-temperature water tank adjusted to 25°C, the viscosity was measured using a Brookfield viscometer (model number: DV1MRVTJ0) at 25°C after 1-minute rotation at a rotation speed of 20 revolutions per minute. For the measurement, rotor No. 3 was used for the viscosity of less than 2000 mPa·s, rotor No. 4 was used for the viscosity of 2000 mPa·s or more and less than 5000 mPa·s, rotor No. 5 was used for the viscosity of 5000 mPa·s or more and less than 15000 mPa·s, rotor No. 6 was used for the viscosity of 15000 mPa·s or more and less than 40000 mPa·s, and rotor No. 7 was used for the viscosity of 40000 mPa·s or more. Samples with a viscosity of preferably 60000 mPa·s or less, more preferably 50000 mPa·s or less, and still more preferably 40000 mPa·s or less are not overly viscous, and have no difficulty in handling.

### Production Example 1

40 g of acrylic acid, 0.22 g of pentaerythritol tetraallyl ether, 0.116 g of 2,2'-azobis(methyl isobutyrate), and 230.9 g of normal hexane were placed in a 500-mL four-necked flask equipped with a stirrer, a thermometer, a nitrogen blowing tube, and a cooling tube. After the solution was stirred and mixed uniformly, nitrogen gas was blown into the solution to remove oxygen present in the upper space of the reaction vessel (four-necked flask), and in the raw material and the reaction solvent. Subsequently, the mixture was heated to 60 to 65°C in a nitrogen atmosphere. The temperature was then maintained at 60 to 65°C for 3 hours. When the mixture was maintained at 60 to 65°C for about one hour, a mixture obtained by dissolving 2.0 g of a block copolymer of 12-hydroxystearic acid and polyoxyethylene (Hypermer B246, produced by Croda) in 2.0 g of normal hexane was added to the reaction vessel. Thereafter, the resulting slurry was heated to 100°C to distill off the normal hexane, and further dried under reduced pressure for 8 hours (115°C, 10 mmHg) to thus obtain 38 g of a white fine powder of a carboxyvinyl polymer. This carboxyvinyl polymer is sometimes referred to below as "the polymer of Production Example 1."

### Production Example 2

40 g of acrylic acid, 0.4 g of BLEMMER VMA-70 (produced by NOF Corporation, a mixture of 10 to 20 parts by mass of stearyl methacrylate, 10 to 20 parts by mass of eicosanyl methacrylate, 59 to 80 parts by mass of behenyl methacrylate, and 1 mass% or less of tetracosanyl methacrylate), 0.19 g of pentaerythritol tetraallyl ether, 0.116 g of 2,2'-azobis(methyl isobutyrate), and 230.9 g of normal hexane were placed in a 500-mL four-necked flask equipped with a stirrer, a thermometer, a nitrogen blowing tube, and a cooling tube. After the solution was stirred and mixed uniformly, nitrogen gas was blown into the solution to remove oxygen present in the upper space of the reaction vessel (four-necked flask), and in the raw material and the reaction solvent. Subsequently, the mixture was heated to 60 to 65°C in a nitrogen atmosphere. The temperature was then maintained at 60 to 65°C for 3 hours. When the mixture was maintained at 60 to 65°C for about one hour, a mixture obtained by dissolving 1.6 g of a block copolymer of 12-hydroxystearic acid and polyoxyethylene (Hypermer B246, produced by Croda) in 2.0 g of normal hexane was added to the reaction vessel. Thereafter, the resulting slurry was heated to 100°C to distill off the normal hexane, and further dried under reduced pressure for 8 hours (115°C, 10 mmHg) to thus obtain 39 g of a white fine powder of an alkyl-modified carboxyl-group-containing water-soluble copolymer. This copolymer is sometimes referred to below as "the polymer of Production Example 2."

### Production Example 3

40 g of acrylic acid, 0.4 g of BLEMMER VMA-70 (produced by NOF Corporation, a mixture of 10 to 20 parts by mass of stearyl methacrylate, 10 to 20 parts by mass of eicosanyl methacrylate, 59 to 80 parts by mass of behenyl methacrylate, and 1 mass% or less of tetracosanyl methacrylate), 0.22 g of pentaerythritol tetraallyl ether, 0.116 g of 2,2'-azobis(methyl isobutyrate), and 230.9 g of normal hexane were placed in a 500-mL four-necked flask equipped with a stirrer, a thermometer, a nitrogen blowing tube, and a cooling tube. After the solution was stirred and mixed uniformly, nitrogen gas was blown into the solution to remove oxygen present in the upper space of the reaction vessel (four-necked flask), and in the raw material and the reaction solvent. Subsequently, the mixture was heated to 60 to 65°C in a nitrogen atmosphere. The temperature was then maintained at 60 to 65°C for 3 hours. When the mixture was maintained at 60 to 65°C for about one hour, a mixture obtained by dissolving 1.6 g of a block copolymer of 12-hydroxystearic acid and polyoxyethylene (Hypermer B246 produced by Croda) in 2.0 g of normal hexane was added to the reaction vessel. Thereafter, the resulting slurry was heated to 100°C to distill off the normal hexane, and further dried at 115°C under 10 mmHg for 8 hours under reduced pressure to thus obtain 39 g of a white fine powder of an alkyl-modified carboxyl-group-containing water-soluble copolymer. This copolymer is sometimes referred to below as "the polymer of Production Example 3."

### Production Example 4

40 g of acrylic acid, 0.22 g of pentaerythritol tetraallyl ether, 0.116 g of 2,2'-azobis(methyl isobutyrate), and 230.9 g of normal hexane were placed in a 500-mL four-necked flask equipped with a stirrer, a thermometer, a nitrogen blowing tube, and a cooling tube. After the solution was stirred and mixed uniformly, nitrogen gas was blown into the solution to remove oxygen present in the upper space of the reaction vessel (four-necked flask), and in the raw material and the reaction solvent. Subsequently, the mixture was heated to 60 to 65°C in a nitrogen atmosphere. The temperature was then maintained at 60 to 65°C for 3 hours. When the mixture was maintained at 60 to 65°C for about one hour, a mixture obtained by dissolving 0.16 g of polyoxyethylene castor oil (produced by Nikko Chemicals Co., Ltd., product name: CO-3, an adduct in which 3 mol of ethylene oxide is added), and 0.60 g of polyoxyethylene hydrogenated castor oil triisostearate (Nihon Emulsion Co., Ltd., product name: RWIS-350, an adduct in which 50 mol of ethylene oxide is added) in 2.0 g of normal hexane was added to the reaction vessel. Thereafter, the resulting slurry was heated to 100°C to distill off the normal hexane, and further dried under reduced pressure for 8 hours (115°C, 10 mmHg) to thus obtain 37 g of a white fine powder of a carboxyvinyl polymer. This carboxyvinyl polymer is sometimes referred to below as "the polymer of Production Example 4."

### Production Example 5

40 g of acrylic acid, 0.88 g of BLEMMER VMA-70 (produced by NOF Corporation, a mixture of 10 to 20 parts by mass of stearyl methacrylate, 10 to 20 parts by mass of eicosanyl methacrylate, 59 to 80 parts by mass of behenyl methacrylate, and 1 mass% or less of tetracosanyl methacrylate), 0.22 g of pentaerythritol tetraallyl ether, 0.116 g of 2,2'-azobis(methyl isobutyrate), and 230.9 g of normal hexane were placed in a 500-mL four-necked flask equipped with a stirrer, a thermometer, a nitrogen blowing tube, and a cooling tube. After the solution was stirred and mixed uniformly, nitrogen gas was blown into the solution to remove oxygen present in the upper space of the reaction vessel (four-necked flask), and in the raw material and the reaction solvent. Subsequently, the mixture was heated to 60 to 65°C in a nitrogen atmosphere. The temperature was then maintained at 60 to 65°C for 3 hours. When the mixture was maintained at 60 to 65°C for about one hour, a mixture obtained by dissolving 0.26 g of polyoxyethylene castor oil (produced by Nikko Chemicals Co., Ltd., product name: CO-3, an adduct in which 3 mol of ethylene oxide is added), and 0.98 g of polyoxyethylene hydrogenated castor oil triisostearate (Nihon Emulsion Co., Ltd., product name: RWIS-350, an adduct in which 50 mol of ethylene oxide is added) in 2.0 g of normal hexane was added to the reaction vessel. Thereafter, the resulting slurry was heated to 100°C to distill off the normal hexane, and further dried under reduced pressure for 8 hours (115°C, 10 mmHg) to thus obtain 38 g of a white fine powder of an alkyl-modified carboxyl-group-containing water-soluble copolymer. This copolymer is sometimes referred to below as "the polymer of Production Example 5."

### Production Example 6

45 g (0.625 mol) of acrylic acid, 0.135 g of pentaerythritol tetraallyl ether, 150 g of n-hexane, and 0.081 g (0.00035 mol) of 2,2'-azobis methyl isobutyrate were placed in a 500-mL four-necked flask equipped with a stirrer, a thermometer, a nitrogen blowing tube, and a cooling tube, to prepare a reaction liquid. After the reaction liquid was stirred and mixed uniformly, nitrogen gas was blown into the solution to remove oxygen present in the upper space of the reaction vessel, and in the raw material and the solvent. Thereafter, the reaction liquid was reacted for 4 hours in a nitrogen atmosphere while the temperature was maintained at 60 to 65°C. After completion of the reaction, the resulting slurry was heated to 90°C to distill off the n-hexane, and further dried under reduced pressure for 8 hours (110°C, 10 mmHg) to thus obtain 42 g of a carboxyvinyl polymer. This carboxyvinyl polymer is sometimes referred to below as "the polymer of Production Example 6."

### Production Example 7

45 g (0.625 mol) of acrylic acid, 0.27 g of pentaerythritol tetraallyl ether, 150 g of n-hexane, and 0.081 g (0.00035 mol) of 2,2'-azobis methyl isobutyrate were placed in a 500-mL four-necked flask equipped with a stirrer, a thermometer, a nitrogen blowing tube, and a cooling tube, to prepare a reaction liquid. After the reaction liquid was stirred and mixed uniformly, nitrogen gas was blown into the solution to remove oxygen present in the upper space of the reaction vessel, and in the raw material and the solvent. Thereafter, the reaction liquid was reacted for 4 hours in a nitrogen atmosphere while the temperature was maintained at 60 to 65°C. After completion of the reaction, the resulting slurry was heated to 90°C to distill off the n-hexane, and further dried under reduced pressure for 8 hours (110°C, 10 mmHg) to thus obtain 42 g of a carboxyvinyl polymer. This carboxyvinyl polymer is sometimes referred to below as "the polymer of Production Example 7."

### Production Example 8

45 g (0.625 mol) of acrylic acid, 1.35 g of BLEMMER VMA-70 (produced by NOF Corporation, a mixture of 10 to 20 parts by mass of stearyl methacrylate, 10 to 20 parts by mass of eicosanyl methacrylate, 59 to 80 parts by mass of behenyl methacrylate, and 1 part by mass or less of tetracosanyl methacrylate), 0.02 g of pentaerythritol tetraallyl ether, 150 g of normal hexane, and 0.081 g (0.00035 mol) of 2,2'-azobis methyl isobutyrate were placed in a 500-mL four-necked flask equipped with a stirrer, a thermometer, a nitrogen blowing tube, and a cooling tube. After the solution was stirred and mixed uniformly, nitrogen gas was blown into the solution to remove oxygen present in the upper space of the reaction vessel (four-necked flask), and in the raw material and the solvent. Then, the mixture was reacted for 4 hours in a nitrogen atmosphere while the temperature was maintained at 60 to 65°C. After completion of the reaction, the resulting slurry was heated to 90°C to distill off the normal hexane, and further dried under reduced pressure for 8 hours (110°C, 10 mmHg) to thus obtain 43 g of a white fine powder of an alkyl-modified carboxyl-group-containing water-soluble copolymer. This copolymer is sometimes referred to below as "the polymer of Production Example 8."

### Preparation of 3% Aqueous Dispersion of Polymer

291 g of ion-exchanged water was measured into a 500-ml plastic beaker equipped with a disperser (diameter: 4 cm, height: 1 cm), and 9 g of each of the polymers of the Production Examples was gradually added while stirring at 1,000 rpm. The time taken from the start of polymer introduction to complete dispersion of the polymer in water was recorded as the "polymer introduction time." After the addition, the stirring speed was set to 2,500 rpm, and stirring was continued for 1 hour to thus obtain a 3% (mass%) aqueous dispersion of polymer.

The faster the polymer is absorbed into water, the shorter the polymer introduction time. A shorter polymer introduction time is preferable to enable more efficient preparation of an aqueous dispersion.

### Method for Preparing 5% Aqueous Dispersion of Polymer

285 g of ion-exchanged water was measured into a 500-ml plastic beaker equipped with four paddle blades with a diameter of 7.5 cm and a width of 1.5 cm. While stirring at 250 rpm, 15 g of each polymer obtained in the Production Examples was gradually added (0.5 to 0.6 g each) with a spatula. As for the addition speed, the next 0.5 to 0.6 g of the polymer was added after the polymer introduced with the spatula was no longer observed on the surface of the water, and the polymer was confirmed to be dispersed in water. The time taken from the start of polymer introduction to complete dispersion of the polymer in water was recorded as the "polymer introduction time." After the polymer was introduced, the stirring speed was set to 760 rpm, and stirring was continued for one hour to thus obtain a 5% aqueous dispersion of polymer.

### Method for Preparing 6% Aqueous Dispersion of Polymer

A 6% aqueous dispersion of polymer was obtained in the same manner as in the method for preparing a 5% aqueous dispersion of polymer, except that the amount of ion-exchanged water was changed to 282 g, and the amount of the polymer was changed to 18 g.

### Method for Preparing 7% Aqueous Dispersion of Polymer

A 7% aqueous dispersion of polymer was obtained in the same manner as in the method for preparing a 5% aqueous dispersion of polymer, except that the amount of ion-exchanged water was changed to 279 g, and the amount of the polymer was changed to 21 g.

### Examples 1 to 11, Comparative Examples 1 to 3, and Reference Example 1

Aqueous dispersions containing each polymer obtained in the Production Examples above in each concentration were prepared based on the formulations shown in Table 1 and according to the method for preparing an aqueous dispersion described above. In Table 1, each numerical value of the components of the formulations of the polymer raw materials and the formulations of the additives are based on parts by mass. In Table 1, each numerical value of the formulation of the aqueous dispersions of polymer is based on percent by mass in each dispersion. Table 1 also shows the viscosity of each aqueous dispersion of polymer, as well as the polymer introduction time during preparation.

**Table 1**

| | Type of polymer | Polymer raw materials | | | Additives*2 | | | Formulation of aqueous dispersions of polymer | | Polymer introduction time (h) | Viscosity (mPa·s) |
|---|---|---|---|---|---|---|---|---|---|---|---|
| | | Acrylic acid | (Meth)aaylic add alkyl ester | PETA*1 | RWIS-350 | Nikkol CO3 | Hypermer B246 | Polymer | Water | | |
| Ex. 1 | Polymer of Production Example 1 | 100 | - | 0.55 | - | - | 5 | 5.0 | 95.0 | 4 | 1,920 |
| Ex. 2 | Polymer of Production Example 1 | 100 | - | 0.55 | - | - | 5 | 6.0 | 94.0 | 6 | 5,940 |
| Ex. 3 | Polymer of Production Example 1 | 100 | - | 0.55 | - | - | 5 | 7.0 | 93.0 | 5 | 18,750 |
| Ex. 4 | Polymer of Production Example 2 | 100 | 1.0 | 0.475 | - | - | 4 | 5.0 | 95.0 | 4 | 2,860 |
| Ex. 5 | Polymer of Production Example 2 | 100 | 1.0 | 0.475 | - | - | 4 | 6.0 | 94.0 | 4 | 11,980 |
| Ex. 6 | Polymer of Production Example 2 | 100 | 1.0 | 0475 | - | - | 4 | 7.0 | 93.0 | 6 | 33,350 |
| Ex. 7 | Polymer of Production Example 3 | 100 | 1.0 | 0.55 | - | - | 4 | 5.0 | 95.0 | 4 | 1,590 |
| Ex. 8 | Polymer of Production Example 3 | 100 | 1.0 | 0.55 | - | - | 4 | 6.0 | 94.0 | 4 | 7,600 |
| Ex. 9 | Polymer of Production Example 3 | 100 | 1.0 | 0.55 | - | - | 4 | 7.0 | 93.0 | 5 | 19,900 |
| Ex. 10 | Polymer of Production Example 4 | 100 | - | 0.55 | 1.5 | 0.4 | - | 5.0 | 95.0 | 30 | 55,600 |
| Ex. 11 | Polymer of Production Example 5 | 100 | 22 | 0.55 | 245 | 0.65 | - | 5.0 | 95.0 | 80 | 24,600 |
| Com. Ex. 1 | Polymer of Production Example 6 | 100 | - | 0.3 | - | - | - | 5.0 | 95.0 | 20 | Non-uniform |
| Com. Ex. 2 | Polymer of Production Example 7 | 100 | - | 0.6 | - | - | - | 5.0 | 95.0 | 10 | Separated (formation of aggregate) |
| Com. Ex. 3 | Polymer of Production Example 8 | 100 | 3.0 | 0.044 | - | - | - | 5.0 | 95.0 | 19 | 148,000 (Sticky like rice cake, impossible to handle) |
| Ref. Ex. 1 | Polymer of Production Example 8 | 100 | 3.0 | 0.044 | - | - | - | 3.0 | 97.0 | 3 | 24,200 |

| | | | | | | | | | | | |
|---|---|---|---|---|---|---|---|---|---|---|---|
| *1: PETA: pentaerythritol tetraallyl ether *2: RWIS-350: polyoxyethylene(50) hydrogenated castor oil triisostearate; Nikkol CO3: polyoxyethylene(3) castor oil; Hypermer B246: block copolymer of 12-hydroxystearic acid and ethylene oxide | | | | | | | | | | | |

### Application Example 1

A thermomassage gel was prepared by using the 5% aqueous dispersion of Example 1 as follows.

89.49 g of glycerol (a special grade reagent, produced by Kanto Chemical Co., Inc.), 1,3-butanediol (a special grade reagent, produced by Kanto Chemical Co., Inc.), and 0.4 g of phenoxyethanol (a special grade reagent, produced by Kanto Chemical Co., Inc.) were measured into a 300-ml plastic beaker equipped with four paddle blades with a diameter of 5 cm and a width of 1.5 cm. The mixture was stirred at room temperature until uniform. Then, 4.0 g of the 5% aqueous dispersion of polymer obtained in Example 1 was added thereto, and the mixture was stirred at 750 rpm for 30 minutes. Thereafter, 1.11 g of a 6% aqueous solution of sodium hydroxide was added, and the mixture was stirred at 1,000 rpm for 30 minutes to obtain a glycerol gel. All of the processes were carried out at room temperature.

The formulation above contained almost no water. Thus, it is necessary to disperse the polymer at a high concentration mainly in water, followed by mixing with glycerol, or to disperse the polymer in glycerol. Dispersing a polymer in glycerol would require stirring over a long period of time, which is not practical. On the other hand, for dispersing a polymer at a high concentration in water (specifically, 5 wt%) as in this Application Example, the mixing of an aqueous dispersion of polymer with glycerol can be performed quickly, which is preferable to reduce the production time. Additionally, the preparation of a high concentration of an aqueous dispersion of polymer can reduce the volume of the tank for the preparation liquid, which is more economical in terms of storage costs and equipment, compared to the case in which a low-concentration aqueous dispersion of polymer is prepared.

The resulting glycerol gel had a viscosity of 26000 mPa·s, and could be easily applied to the skin without spilling. Further, this glycerol gel had a high transparency with a transmittance of 98.5%T, and also had a desirable appearance. When applied to the skin, the moisture on the skin and the heat of hydration of glycerol gave a warm feeling. Thus, this gel was suitable for massage use.

## Claims

1. An aqueous dispersion composition comprising 4 to 8 mass% of a copolymer obtained by polymerization in the presence of a nonionic surfactant with one or more polyoxyethylene chains, the copolymer being at least one member selected from the group consisting of
(1) copolymers obtained by polymerizing the following (i) and (ii),
(2) copolymers obtained by polymerizing the following (i) and (iii), and
(3) copolymers obtained by polymerizing the following (i), (ii), and (iii):
(i) 100 parts by mass of a (meth)acrylic acid;
(ii) 0.001 to 1 part by mass of a compound with two or more ethylenically unsaturated groups; and
(iii) 0.5 to 5 parts by mass of a (meth)acrylic acid alkyl ester having an alkyl group with 10 to 30 carbon atoms.

2. The aqueous dispersion composition according to claim 1, wherein the copolymer is obtained by polymerization in the presence of a nonionic surfactant with one or more polyoxyethylene chains in an amount of 0.5 to 10 parts by mass per 100 parts by mass of the (meth)acrylic acid (i).

3. The aqueous dispersion composition according to claim 1 or 2, having a viscosity of 60000 mPa·s or less.

4. The aqueous dispersion composition according to any one of claims 1 to 3, wherein the nonionic surfactant with one or more polyoxyethylene chains is selected from the group consisting of polyhydric alcohol fatty acid ester-ethylene oxide adducts, block copolymers of hydroxy fatty acids and ethylene oxide, and polyoxyethylene castor oil.

5. An aqueous dispersion composition comprising 4 to 8 mass% of at least one member selected from the group consisting of
(1) copolymers obtained by polymerizing the following (i) and (ii),
(2) copolymers obtained by polymerizing the following (i) and (iii), and
(3) copolymers obtained by polymerizing the following (i), (ii), and (iii), and
having a viscosity of 60000 mPa·s or less:
(i) 100 parts by mass of a (meth)acrylic acid;
(ii) 0.001 to 1 part by mass of a compound with two or more ethylenically unsaturated groups; and
(iii) 0.5 to 5 parts by mass of a (meth)acrylic acid alkyl ester having an alkyl group with 10 to 30 carbon atoms.

6. The aqueous dispersion composition according to any one of claims 1 to 5, wherein the (meth)acrylic acid alkyl ester (iii) is a (meth)acrylic acid alkyl ester having an alkyl group with 18 to 24 carbon atoms.

7. The aqueous dispersion composition according to any one of claims 1 to 6, wherein the compound with two or more ethylenically unsaturated groups (ii) is at least one compound selected from the group consisting of pentaerythritol allyl ether, tetraallyloxyethane, triallyl phosphate, and polyallylsaccharose.
